# EUROPEAN PATENT SPECIFICATION

(11) **EP 0 938 339 B1**
(45) Date of publication and mention of the grant of the patent: **10.07.2002**
(21) Application number: 97943856.1
(22) Date of filing: 10.09.1997
(51) Int. Cl.: A61K 47/22, A61K 47/44

(54) **FORMULATIONS FOR TOPICAL USE CONTAINING VIT. E ACETATE**
PHARMAZEUTISCHE FORMULIERUNGEN ZUR TOPISCHEN VERWENDUNG MIT VIT. E ACETATE
FORMULATIONS DESTINEES A UN USAGE TOPIQUE A BASE DE VIT. E ACETATE

(30) Priority: 11.09.1996 IT MI961865
(43) Date of publication of application: 01.09.1999
(73) Proprietor: BIO.LO.GA. S.r.l., 31015 Conegliano (TV) (IT)
(72) Inventor: Panin, Giorgio, 45100 Rovigo (IT)
(74) Representative: Ferreccio, Rinaldo
(86) International application number: EP9704946
(87) International publication number: WO9810793

(56) References cited:
- EP-A- 0 258 558
- WO-A-94/01088
- US-A- 4 551 332
- CHEMICAL ABSTRACTS, vol. 118, no. 22, 31 May 1993 Columbus, Ohio, US; abstract no. 219507, XP002051582 & JP 05 025 030 A (KURABU KOSUMECHITSUKUSU KK,JP) 2 February 1993
- DATABASE WPI Week 8349 Derwent Publications Ltd., London, GB; AN 83-835500 [25] XP002051583 & JP 58 183 938 A (SHISEIDO) 27 October 1983

## Description

The present invention relates to the field of pharmaceutical technology.

In particular, the invention relates to novel formulations for topical use, having a liquid or semisolid consistency, that are to be applied to the skin.

Depending on the physical system by which they are constituted, such formulations are subdivided into grease, ointments, creams, gels and pastes.

Greases, in their turn, are subdivided into oils and ointments and the latter, in their turn, are subdivided into hydrophobic ointments, water-absorbing ointments and hydrophilic ointments.

Among creams, a distinction is made between hydrophobic creams, hydrophilic creams and amphiphilic creams, and among gels a distinction is made between hydrophobic gels and hydrophilic gels.

The above-mentioned topical formulations constitute the basis of officinal galenica, that is to say, formulations of constant composition ready for use as such in pharmacy, of magistral galenica and of many medical specialities for topical use.

With the exception of hydrophilic ointments and hydrophilic gels, all the formulations mentioned above contain a lipophilic phase, generally composed of petroleum jelly, paraffin and mineral oils, to which emulsifiers are optionally added to permit the incorporation of active ingredients dissolved in aqueous phases.

However, the above-mentioned substances are hydrocarbons derived from petroleum, which, even if subjected to purification processes, still contain contaminating substances, and especially polycyclic aromatic hydrocarbons which are regarded as being responsible for some forms of contact dermatitis and as having a carcinogenic action.

Furthermore, the topical formulations based on the above-mentioned lipophilic substances, and especially creams and hydrophobic ointments, have the major disadvantage of leaving the skin greasy and of imparting to the user a feeling of having foreign matter on his or her skin, giving rise to a need for cleansing.

The aim of the present invention is to provide a topical formulation which overcomes the disadvantages discussed above with reference to the products of the prior art.

This aim has been achieved by means of a formulation for topical use comprising a lipophilic phase, characterised in that it includes vitamin E (dl-alpha-tocopherol) or a pharmaceutically acceptable ester thereof amongst the components of the lipophilic phase.

Preferably, the topical formulation according to the invention includes vitamin E acetate (dl-alpha-tocopherol) amongst the components of the lipophilic phase.

Naturally, it is also possible to use 1-alpha-tocopherol acetate and d-alpha-tocopherol acetate instead of dl-alpha-tocopherol acetate.'

The lipophilic phase of the formulation generally comprises from 20 to 100% by weight of vitamin E acetate, preferably from 30 to 100%, most preferably from 51 to 100% by weight, optionally in admixture with animal, vegetable or synthetic fats and oils, mineral oils and silicone oils.

Among the animal and vegetable fats and oils with which the vitamin E acetate can be mixed, there may be mentioned beeswax, lanolin and its derivatives, olive oil, almond oil, sesame oil, coconut oil, groundnut oil, jojoba oil, avocado oil, karite butter and, as mineral oils, liquid paraffin, petroleum jelly, silicone oil, volatile silicones.

If it is desired to modify the consistency or the viscosity of the topical formulations prepared using vitamin E acetate, it is possible to add to the vitamin E acetate one or more vegetable or mineral oils or waxes and long-chained aliphatic alcohols, such as, for example, stearyl alcohol and cetostearyl alcohol, or, in the case of hydrophobic gels, polyethylene, colloidal silicon dioxide, Zn or Al soaps and hydrogenated castor oil.

In order to permit the preparation of galenic formulations containing hydrophilic active substances, it is possible to add to the vitamin E acetate or to a mixture thereof with other oils or fats, a surfactant, such as, for example, sodium lauryl sulphate, cetomacrogol, monoglycerides or other, similar, substances customarily used in the basic ointments of the various pharmacopoeias.

If it is desired to minimise the risk of allergic reaction as far as possible, it is advisable to use the vitamin E acetate as the only component of the lipophilic phase of the formulations according to the invention.

The possibility of using vitamin E acetate as the principal component of the lipophilic phase of topical formulations is somewhat surprising because such a use has never even been suggested in the prior art.

Vitamin E and its derivatives are substances widely used in the pharmaceutical and cosmetic industry, owing to their alleged anti-oxidant properties and properties of removing free radicals, in the preparation of formulations for healing skin disorders or for combating or preventing unsightly skin imperfections.

For those reasons, vitamin E and its derivatives are often present in cosmetic formulations as "active" components in concentrations varying from 1 to 25%, although, in fact, the anti-oxidant and anti-ageing action of vitamin E on the skin has never been demonstrated scientifically.

However, it has now been found experimentally that vitamin E acetate has chemico-physical properties rendering it suitable for use as the principal component of the lipophilic phase of topical formulations.

Vitamin E acetate can be spread reasonably easily, is absorbed with surprising rapidity, does not give rise to unpleasant sensations of heat, leaves the skin shiny only for a few minutes and then soft, elastic and non-sticky and is resistant to cleansing with water or detergents.

In addition, owing to the fact that vitamin E acetate is not a molecule foreign to the human organism, 'it can readily be integrated in the lipids present in the stratum corneum and can facilitate absorption of substances dispersed therein through the skin.

In order to evaluate the absorption of vitamin E acetate, subjective tests were carried out on 20 individuals aged from 25 to 60.

The application of vitamin E acetate was pleasant owing to the absence of any odour, owing to the feel of skin softness, the absence of greasiness, the absence of any sensation of applied foreign matter and, consequently, the absence of a need for cleansing, the lack of side effects during absorption (no stinging of the skin, no sensation of the stabbing type, even where the skin was irritated).

Two minutes after the application of vitamin E acetate, the skin did not exhibit any residual greasiness.

Using vitamin E acetate as the principal component of the lipophilic phase of topical formulations, various formulations exhibiting optimum application, absorption and thermal stability characteristics were prepared for topical use.

The following formulations, in which the percentages of the individual components are by weight based on the total weight of the formulation, are given by way of example :

| Ointment containing cetomacrogol, which is not part of the invention. | |
|---|---|
| dl-alpha-tocopherol acetate | 70% |
| cetostearyl alcohol | 24% |
| cetomacrogol | 6% |

The ointment is prepared by heating the components together until they melt and agitating until cooling has occurred.

| Sodium lauryl sulphate ointment base, which is not part of the invention. | |
|---|---|
| dl-alpha-tocopherol acetate | 70% |
| cetostearyl alcohol | 27% |
| sodium lauryl sulphate | 3% |

The ointment is prepared by heating the components together until they melt and agitating until cooling has occurred.

| Cream base containing cetomacrogol (O/W), which is not part of the invention. | |
|---|---|
| dl-alpha-tocopherol acetate | 21% |
| cetostearyl alcohol | 7.2% |
| cetomacrogol (1000) | 1.8% |
| purified water | 70% |

The cream is prepared by melting the solid components in the dl-alpha-tocopherol acetate and adding freshly boiled water at the same temperature, agitating slowly until cooling has occurred.

| Fatty cream base containing cetomacrogol (W/O), which is not part of the invention. | |
|---|---|
| dl-alpha-tocopherol acetate | 39% |
| cetostearyl alcohol | 18% |
| cetomacrogol (1000) | 4.5% |
| purified water | 25% |

The cream is prepared by melting the solid components in the dl-alpha-tocopherol 'acetate and adding freshly boiled water at the same temperature, agitating slowly until cooling has occurred and reintegrating any evaporated water.

| Oily cream, which is not part of the invention | |
|---|---|
| dl-alpha-tocopherol acetate | 60% |
| cetyl palmitate | 7% |
| white wax | 6% |
| glyceryl monostearate | 2% |
| purified water | 25% |

The above composition is a lipophilic cream base W/O and corresponds to the "oily fatty cream base" of the Codex of conventional galenic preparations of magistral derivation, from which it differs by the substitution of groundnut oil by dl-alpha-tocopherol acetate.

The cream is prepared by heating the dl-alpha-tocopherol acetate, the cetyl palmitate, the glyceryl monostearate and the white wax to approximately 60°C and adding to the molten mass so obtained, at the same temperature, freshly boiled water, agitating slowly until cooling has occurred.

| Amphiphilic cream base, which is not part of the invention. | |
|---|---|
| dl-alpha-tocopherol acetate | 20% |
| white petroleum jelly | 9% |
| liquid semisynthetic triglycerides | 4% |
| cetostearyl alcohol | 6% |
| glyceryl monostearate | 4% |
| polyethylene glycol stearate | 7% |
| propylene glycol | 10% |
| purified water | 40% |

The cream is prepared by heating the dl-alpha-tocopherol acetate, the petroleum jelly, the triglycerides, the cetostearyl alcohol and the glyceryl monostearate to approximately 60°C until melting takes place. The other components are dissolved in freshly boiled water and the solution obtained, heated to approximately 60°C, is combined with the molten mass, agitating until cooling has occurred and optionally integrating any evaporated water.

### Hydrophobic gels

a)

| | |
|---|---|
| dl-alpha-tocopherol acetate | 20% |
| cyclomethicone | 64% |
| dimethiconol | 16% |

The gel is prepared by dispersing the dl-alpha-tocopherol acetate in a preformed mixture of cyclomethicone:dimethiconol 8:2 w/w.
b)

| | |
|---|---|
| dl-alpha-tocopherol acetate | 30% |
| cyclomethicone | 52% |
| dimethiconol | 13% |
| hydrogenated castor oil | 5% |

The gel is prepared by adding dl-alpha-tocopherol acetate to a previously prepared mixture of dimethiconol and cyclomethicone and then dispersing the hydrogenated castor oil in the mixture thus obtained.

Below are two examples of officinal galenic formulations in which vitamin E acetate is used instead of the mineral oils provided for in the corresponding formulations of the pharmacopoeias.

| Paste containing zinc oxide | |
|---|---|
| dl-alpha-tocopherol acetate | 70% |
| zinc oxide | 30% |

The consistency of this formulation can be varied by altering the percentages of the components within a range of from 50 to 85% in the case of dl-alpha-tocopherol acetate and from 15 to 50% in the case of zinc oxide, bearing in mind that an increase in the content of zinc oxide involves an increase in the consistency of the paste.

Compared with the zinc oxide pastes of the official pharmacopoeias, which contain mineral oils, the paste prepared using vitamin E acetate as excipient has a lesser greasiness and is safer to use owing to the absence of mineral oils.

| Ointment containing salicylic acid: | |
|---|---|
| dl-alpha-tocopherol acetate | 95.0-99.9% |
| salicylic acid | 0.1-5.0% |

In this case too, the ointment so prepared exhibits a lesser greasiness and a lower risk of toxicity or allergenicity than do conventional salicylic acid ointments containing mineral oils.

In addition, the keratolytic action of the ointment is accompanied by a lesser irritating effect on the skin than that observed with the conventional salicylic acid ointments.

| Ointment containing clotrimazole | |
|---|---|
| dl-alpha-tocopherol acetate | 99.0% |
| clotrimazole | 1.0%. |

The ointment so prepared exhibited a very good absorption rate and the antifungal effect of clotrimazole resulted to be enhanced and accelerated with respect to the topical formulations so far available on the market.

Among the active ingredients for which the suitability of the topical formulations according to the 'invention as excipients was checked experimentally, there may be mentioned, purely by way of example, antibiotics, such as gentamicin, neomycin, clindamycin and tetracyclines, disinfectants, such as chlorhexidine, corticosteroids, such as hydrocortisone acetate or butyrate, diflucortolone valerate, methylprednisolone aceponate, mometasone furoate, and esters of betametasone, antimycotic agents, such as e.g. clotrimazole, miconazole, ketoconazole, econazole, tolnaftate, topical anti-inflammatory agents, such as nimesulide, ibuprofen, benzydamine and bendazac, trans-retinoic acid, calcipotriol, vitamins such as retinol and its derivatives (retinol acetate and palmitate), lipophilic derivatives of ascorbic acid, such as palmitoylascorbic acid, vitamin k, vitamin D, escin and capillary protectants, such as quercetin, rutin and flavonoids.

Pharmaceutical formulations for topical use based on vitamin E acetate and containing up to.3% of the above-mentioned antibiotics, other formulations containing up to 0.1% of trans-retinoic acid, formulations containing up to 0.005% of calcipotriol, formulations containing up to 700000 IU% of retinol, formulations containing up to 2% clotrimazole and finally formulations containing up to 1% of hydrocortisone acetate or butyrate or of diflucortolone valerate were prepared.

All of the formulations mentioned above were found to exhibit optimum stability in respect of heat and time and rapid absorption both of the excipient and of the active ingredient dispersed therein.

A pharmaceutical formulation in cream for topical use containing, as active ingredients, diflucortolone valerate and chlorquinaldol is given hereinafter purely by way of example.

| | |
|---|---|
| diflucortolone valerate | 0.1% |
| chlorquinaldol | 1% |
| dl-alpha-tocopherol acetate | 20% |
| stearyl alcohol | 8% |
| PEG monostearate | 3% |
| disodium EDTA | 0.1% |
| carboxypolymethylene | 0.3% |
| NaOH | 0.07% |
| purified water | 67.43% |

The cream is prepared by moistening the carboxypolymethylene with 10 parts of water and then by adding the rest of the water, agitating until a dispersion free from lumps is obtained. The disodium EDTA and the PEG stearate are then added, with agitation, to the dispersion, while heating at approximately 70°C.

The dispersion so obtained is combined, by mixing, with a mixture of dl-alpha-tocopherol acetate and stearyl alcohol previously heated to approximately 60°C.

The sodium hydroxide is added in the form of a 10% p/v solution, with agitation, to the emulsion obtained, reintegrating any water that has evaporated and continuing to agitate until cooling has occurred.

Finally, dl-alpha-tocopherol acetate may be used as a component of sunscreen products instead of most or all the animal, vegetable or mineral oils therein contained. An example of a sunscreen product containing vitamin E acetate as the main lipophilic component is the following:

| | |
|---|---|
| dl-alpha-tocopherol acetate | 20% |
| p-aminobenzoic acid | 5% |
| stearic acid | 3% |
| propylene glycol | 2.9% |
| cetyl alcohol | 0.5% |
| triethanolamine | 0.5%. |
| methyl and propyl paraben 5:1 | 0.25% |
| perfume | 0.25% |
| water | 67.6% |

In addition to p-aminobenzoic acid, all the other usual sunscreens can be used in the sunscreen formulations containing vitamin E acetate as the main lipophilic component: for instance chemical sunscreens such as p-aminobenzoic acid esters, benzophenone derivatives, cinnamic acid derivatives, benzylidenecamphor derivatives, salicylate derivatives, and physical. sunscreens such as titanium dioxide and zinc oxide.

Vitamin E acetate can suitably be used for sunscreens formulation of any nature: lotions, creams, sprays etc..

## Claims

1. A formulation for topical use which comprises a lipophilic phase including vitamin E acetate and which is in the form of a hydrophobic gel, **characterized in that** the lipophilic phase further comprises a mixture of cyclomethicone and dimethiconol.

2. A formulation according to claim 1, wherein the amount of vitamin E acetate is at least 20% by weight.

3. A formulation according to any of claims 1 and 2, **characterized in that** cyclomethicone and dimethiconol are contained in said mixture in a weight ratio of 8:2.

4. A formulation according to any one of claims 1 to 3, further comprising hydrogenated castor oil.

5. A pharmaceutical formulation for topical use comprising at least one pharmaceutically active substance and an excipient which is constituted by a formulation according to any one of claims 1 to 4.

6. A pharmaceutical formulation according to claim 5, wherein said active substance is selected among zinc oxide, salicylic acid and clotrimazole

7. A pharmaceutical formulation according to claim 6, which consists of 15 to 49% by weight of zinc oxide and of 51 to 85% by weight of vitamin E acetate.

8. A pharmaceutical formulation according to claim 6, which comprises salicylic acid.

9. A pharmaceutical formulation according to claim 6, which comprises up to 2% by weight of clotrimazole.

10. Non-therapeutic of a formulation according to anyone of claims 1 to 4 as a sunscreen product.

## Patentansprüche

1. Formulierung zur örtlichen Verwendung, die eine lipophile Phase aufweist, die Vitamin E-acetat enthält, und die in der Form eines hydrophoben Gels vorliegt, **dadurch gekennzeichnet, daß** die lipophile Phase außerdem ein Gemisch aus Cyclometicon und Dimeticonol aufweist.

2. Formulierung nach Anspruch 1, bei der die Menge an Vitamin E-acetat mindestens 20 Gew.-Prozent beträgt.

3. Formulierung nach Anspruch 1 oder 2, **dadurch gekennzeichnet, daß** Cyclometicon und Dimeticonol in dem Gemisch in einem Gewichtsverhältnis von 8:2 enthalten sind.

4. Formulierung nach einem der Ansprüche 1 bis 3, außerdem hydriertes Castor-öl aufweisend.

5. Pharmazeutische Formulierung zur örtlichen Verwendung, aufweisend mindestens einen pharmazeutischen Wirkstoff und eine Trägersubstanz, die gebildet wird von einer Formulierung nach einem der Ansprüche 1 bis 4.

6. Pharmazeutische Formulierung nach Anspruch 5, bei der der Wirkstoff ausgewählt ist unter Zinkoxid, Salicylsäure und Clotrimazol.

7. Pharmazeutische Formulierung nach Anspruch 6, die besteht aus 15 bis 49 Gew.-% Zinkoxid und 51 bis 85 Gew.-% Vitamin E-acetat.

8. Pharmazeutische Formulierung nach Anspruch 6, die Salicylsäure aufweist.

9. Pharmazeutische Formulierung nach Anspruch 6, die bis zu zwei Gew.-% Clotrimazol aufweist.

10. Nicht-therapeutische Verwendung einer Formulierung nach einem der Ansprüche 1 bis 4 als ein Sonnenschutzmittel.

## Revendications

1. Une formulation pour usage topique, qui comprend une phase lipophile comprenant de la vitamine E acétate et qui est sous forme d'un gel hydrophobe, **caractérisée en ce que** la phase lipophile comprend en outre un mélange de cyclométhicone et de diméthiconol.

2. Une formulation selon la revendication 1, où la quantité de vitamine E acétate est d'au moins 20 % en poids.

3. Une formulation selon l'une des revendications 1 et 2, **caractérisée en ce que** le cyclométhicone et le diméthiconol sont contenus dans ledit mélange dans un rapport pondéral de 8:2.

4. Une formulation selon l'une des revendications 1 à 3, comprenant en outre de l'huile de castor hydrogénée.

5. Une formulation pharmaceutique pour usage topique, comprenant au moins une substance pharmaceutiquement active et un excipient qui est constitué par une formulation selon l'une des revendications 1 à 4.

6. Une formulation pharmaceutique selon la revendication 5, où ladite substance active est choisie parmi l'oxyde de zinc, l'acide salicylique et le clotrimazole.

7. Une formulation pharmaceutique selon la revendication 6, qui consiste en 15 à 49 % en poids d'oxyde de zinc et 51 à 85 % en poids de vitamine E acétate.

8. Une formulation pharmaceutique selon la revendication 6, qui comprend de l'acide salicylique.

9. Une formulation pharmaceutique selon la revendication 6, qui comprend jusqu'à 2 % en poids de clotrimazole.

10. Utilisation non thérapeutique d'une formulation selon l'une des revendications 1 à 4 comme produit écran solaire.
